Europäisches Patentamt

European Patent Office

Office européen des brevets

(11) Publication number: **0 235 514**
**A1**

# EUROPEAN PATENT APPLICATION

(21) Application number: 87100497.4

(51) Int. Cl.4: **C07C 59/105** , **B01D 9/02**

(22) Date of filing: 16.01.87

(30) Priority: 21.01.86 JP 11402/86

(43) Date of publication of application:
09.09.87 Bulletin 87/37

(84) Designated Contracting States:
BE CH DE FR GB IT LI NL SE

(71) Applicant: Fujisawa Pharmaceutical Co., Ltd.
3, Doshomachi 4-chome Higashi-ku
Osaka-shi, Osaka 541(JP)

(72) Inventor: Teraji, Tsutomu
6-20-6, Kofudai Toyono-cho Toyono-gun
Osaka-fu(JP)
Inventor: Takahashi, Satoru
6-32-305, Fukazucho
Nishinomiya-shi(JP)
Inventor: Yamamura, Atsushi
3-19-3, Nishifukui
Ibaraki-shi(JP)

(74) Representative: Türk, Dietmar, Dr. rer. nat. et
al
Türk, Gille + Hrabal Patentanwälte Bruckner
Strasse 20
D-4000 Düsseldorf 13(DE)

(54) New N-typed crystals of calcium gluconate.

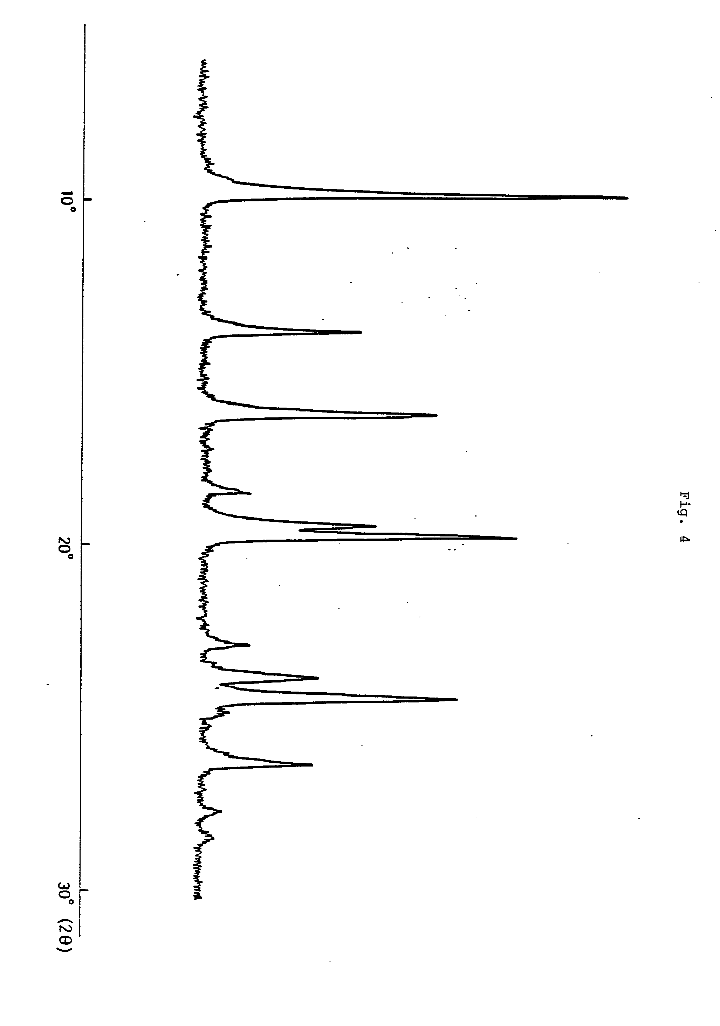

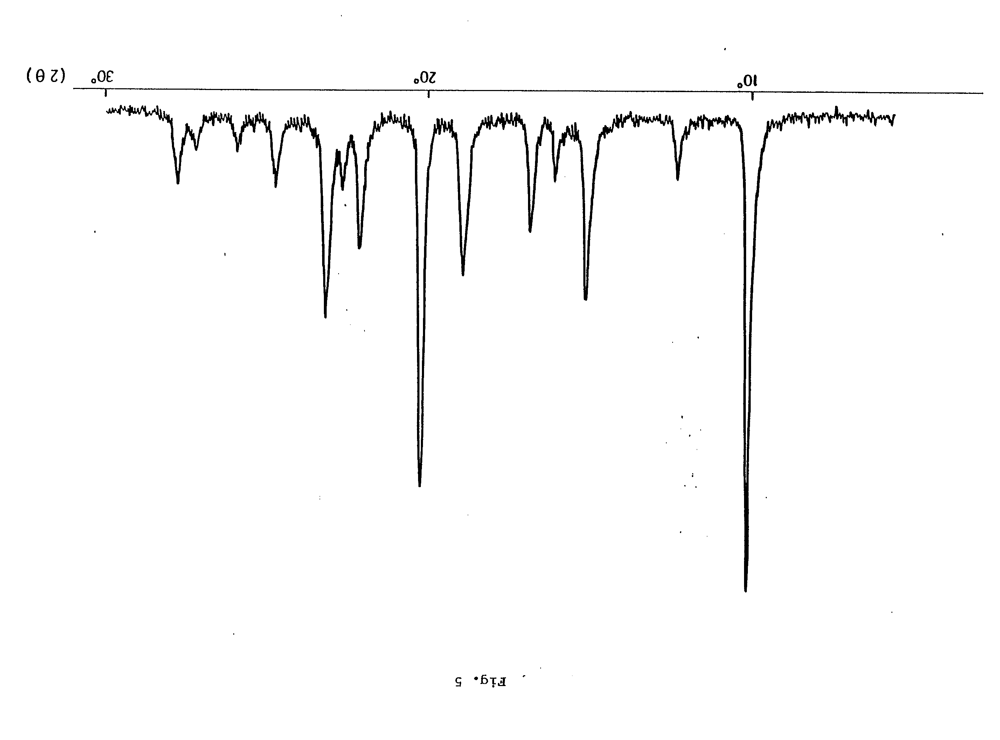

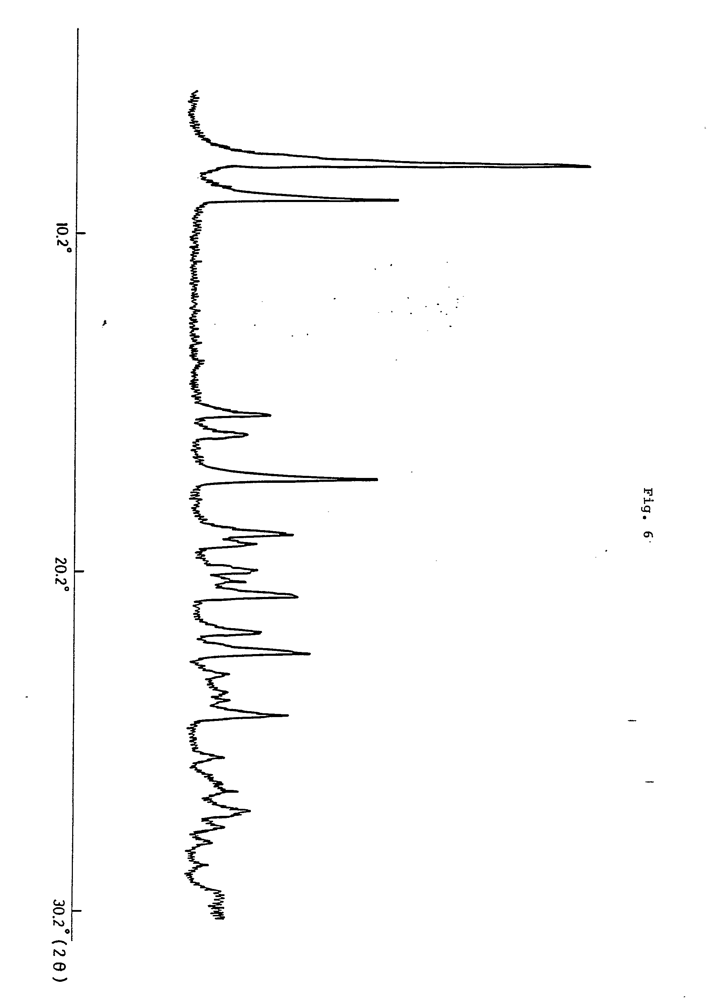

(57) N-typed crystal of calcium gluconate having a powder X-ray diffraction pattern as shown in Fig. 4 and a process for the preparation thereof which comprises precipitating calcium gluconate from an aqueous conventional calcium gluconate solution having the property that conventional crystals of calcium gluconate are not precipitated therefrom at around 40°C, at below 20°C.

Fig. 1

EP 0 235 514 A1

## NEW N-TYPED CRYSTALS OF CALCIUM GLUCONATE

The present invention relates to new N-typed crystals of calcium gluconate. More particularly, it relates to new N-typed crystals of calcium gluconate which are superior to the conventional crystals in solubility and rate of dissolution in water, and to a processes for preparation thereof.

Calcium gluconate has been used widely in pharmaceutical calcium preparations and, also, as a food additive for calcium enrichment, and it is known that the conventional calcium gluconate may exist in two forms of anhydrate and monohydrate.

The solubility of the conventional calcium gluconate ranks comparatively high among organic acid salts but is not as high as desired and for a broader application of calcium gluconate, it is desirable to improve not only its solubility but also its rate of dissolution.

To overcome the above disadvantage, the present inventors conducted an intensive research and found that by precipitating calcium gluconate from an aqueous conventional calcium gluconate solution having the property that conventional crystals of calcium gluconate are not precipitated therefrom at around 40°C, at below 20°C instead of, and avoiding, precipitation from a high temperature solution, there could be obtained N-typed crystals (N-crystals), a novel crystal form, of calcium gluconate which are superior to the conventional calcium gluconate crystals in solubility and rate of dissolution. The present invention has been accomplished on the basis of the above finding.

The N-crystals of calcium gluconate according to the present invention can be produced for example by the following method. The calcium gluconate produced by the conventional technique is dissolved in water, preferably under heating at 70 to 80°C and, either after filtration at about 40°C, which is performed for preventing contamination of the product with the conventional crystals of calcium gluconate, or bypassing such a filtration step, if conventional crystals of calcium gluconate are not precipitated from the resulting solution at around 40°C, the solution is allowed to stand at below 20°C, preferably in a refrigerator. The crystals which separate out thereupon are collected by filtration and dried to give the desired N-crystals.

Alternately, N-crystals of calcium gluconate according to the present invention can be produced by the following procedure. The gluconic acid or gluconodeltalactone prepared by the conventional technique is dissolved in water and after addition of calcium carbonate, the solution is stirred with heating (for example at 95°C for 1 hour). The reaction mixture is cooled to about 40°C and either after filtration or bypassing the step, if conventional crystals of calcium gluconate are not precipitated from the resulting solution at around 40°C, the solution is further cooled to about 13-15°C. Thereafter, N-crystals of calcium gluconate prepared by the method described above are added as seeds and the mixture is stirred at the same temperature for a few hours. The resulting crystals are collected by filtration and dried to obtain N-crystals of calcium gluconate.

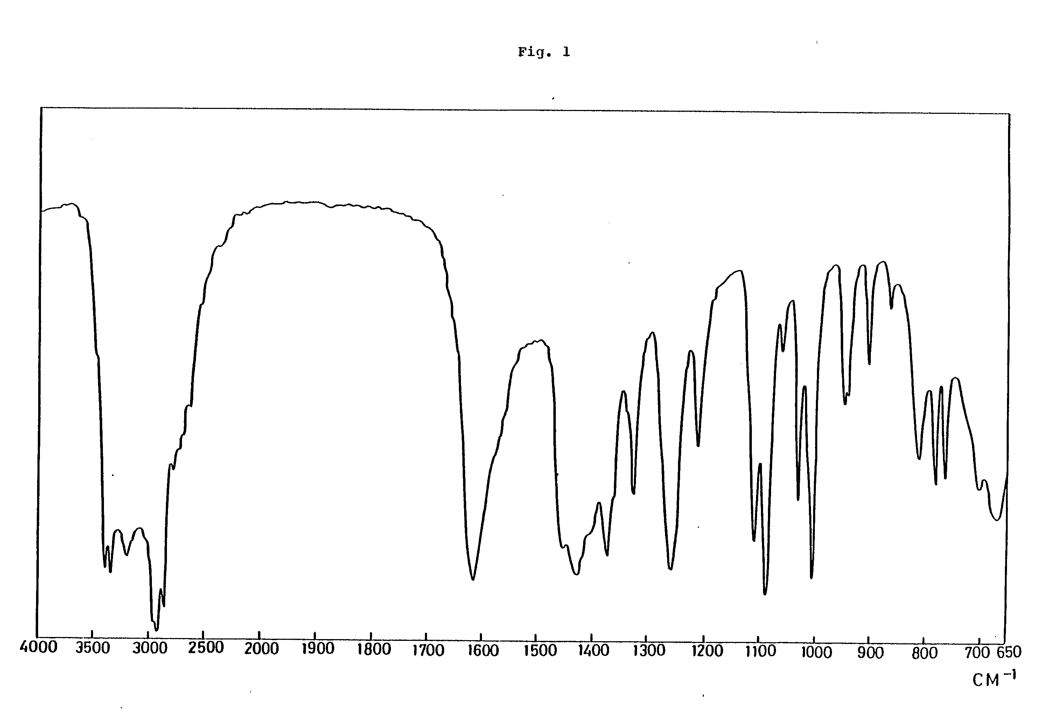

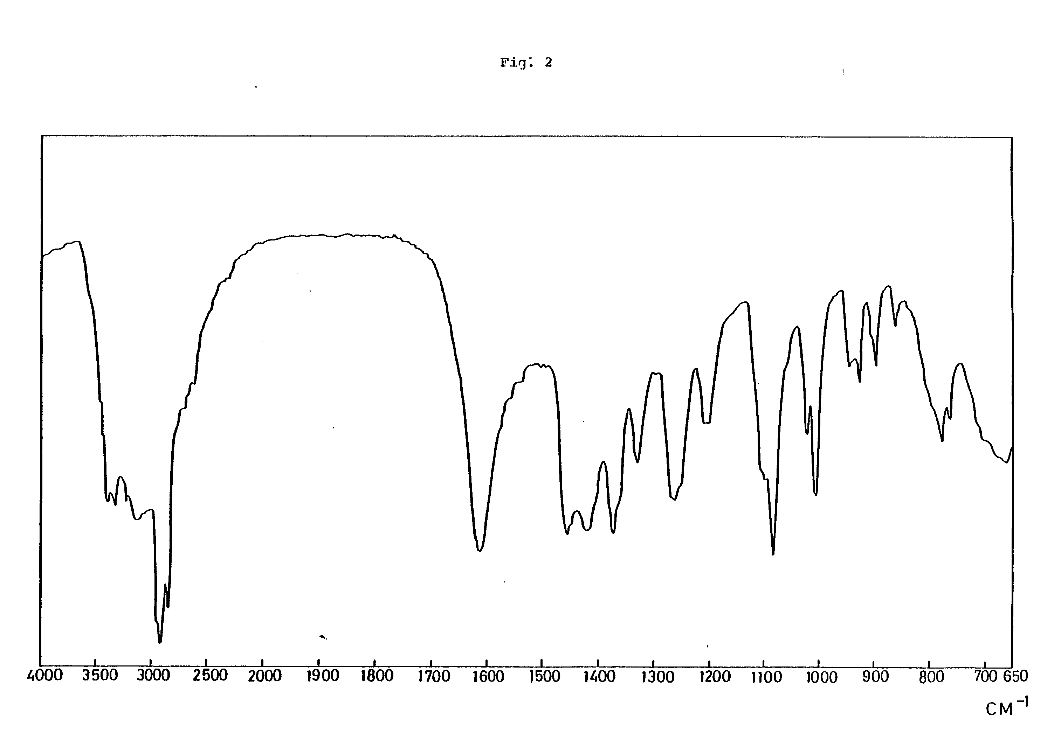

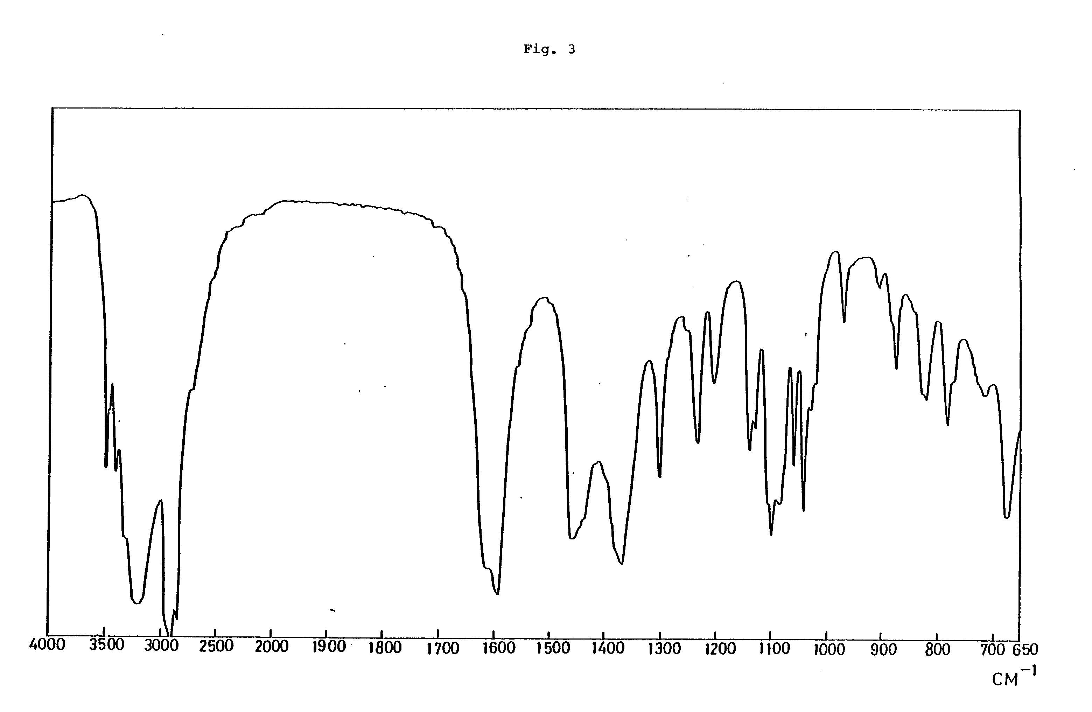

Since these N-crystals show no endothermic peak up to 233°C in differential thermal analysis, they are obviously anhydrate crystals. The infrared absorption spectrum (Nujol) of this product is shown is Fig. 1. - (The infrared absorption spectra of the conventional calcium gluconate anhydrate and monohydrate are shown in Figs. 2 and 3, respectively.) The powder X-ray diffraction pattern of the calcium gluconate N-crystal according to the present invention is shown below in comparison with that of the conventional calcium gluconate.

Powder X-ray diffraction analysis:

(1) Measuring conditions

Counter cathode : copper
Filter : nickel
Tube voltage : 30 KVP
Tube current : 10 mA
Full scale range : 2000 C/S
Time constant : 0.5 sec
Scan speed : 2°/minute
Scan range : $2\theta$ 5-30°
Chart speed : 2 cm/minute
Divergence slit : 1°

Incident beam slit : 0.15 mm
Scatter slit : 1°
Detector : Scincillation counter

(2) Results of determination

| Calcium gluconate N-crystal of the invention (Fig. 4) 2 θ | Conventional calcium gluconate crystal (anhydrate) (Fig. 5) 2 θ | Conventional calcium gluconate crystal (mono-hydrate) (Fig. 6) 2 θ |
|---|---|---|
| 9.8° (strong) | 10.1 (strong) | 8.0 (strong) |
| 13.7 | 12.3 | 9.0 |
| 16.2 | 15.1 | 15.4 |
| 19.4 | 16.0 | 16.0 |
| 19.7 (strong) | 16.8 | 17.3 |
| 22.8 | 18.9 | 18.9 |
| 23.7 | 20.2 (strong) | 19.2 |
| 24.3 (strong) | 22.1 | 20.0 |
| 26.3 | 22.7 | 20.4 |
| | 23.1 | 20.8 |
| | 24.7 | 21.9 |
| | 25.9 | 22.5 |
| | 27.2 | 23.1 |
| | 27.8 | 23.7 |
| | | 23.9 |
| | | 24.3 |
| | | 25.6 |
| | | 27-30 |

As is clear from the test examples given below, the N-crystals of calcium gluconate according to the present invention are superior to the conventional crystals in solubility and rate of dissolution in water and may not only be used as readily soluble calcium gluconate in medical applications but are useful as a food additive as well.

Test Example 1 (Solubility)

A 100 ml conical flask was filled with water (30 ml) followed by addition of an excess of the calcium gluconate indicated below. In a water bath maintained at a predetermined temperature, the contents were stirred with a stirrer for 30 minutes, after which time they were filtered. The amount of calcium gluconate in the filtrate was then determined by titration of the calcium ion.

The results are shown below in Table 1. The figures stand for the amounts in w/w % of calcium gluconate in the respective aqueous calcium gluconate solutions.

## Table 1

| Temperature (°C)<br>Calcium<br>gluconate | 10 | 30 | 50 |
|---|---|---|---|
| Calcium gluconate N-crystals of the Invention | 5.37 | 7.72 | 10.84 |
| Conventional calcium gluconate crystals (anhydrate) | 2.42 | 3.65 | 5.70 |
| Conventional calcium gluconate crystals (monohydrate) | 2.76 | 4.55 | 7.79 |

Test Example 2 (Rate of dissolution)

A 1-liter round-bottomed flask was filled with 300 ml of water and with stirring at a predetermined temperature, calcium gluconate was added at a predetermined varying concentration (w/w %). The time fill dissolution of the calcium gluconate was determined.

The results are set forth in Table 2.

4

## Table 2

| Temperature (°C) | 40 | 50 | 60 |
|---|---|---|---|
| Concentration (w/w %) | 4 | 5 | 6 |
| Calcium gluconate | | | |
| Calcium gluconate N-crystals of the Invention | 2'38" | 1'50" | 1'10" |
| Conventional calcium gluconate crystals (anhydrate) | 69'00" | 26'00" | 13'34" |
| Conventional calcium gluconate crystals (monohydrate) | 24'33" | 13'29" | 3'48" |

Working examples of the present invention are as follows.

Example 1

A 200 ml conical flask was filled with ion-exchanged water (90 ml) and the conventional calcium gluconate (anhydrate) (10 g) was dissolved with heating at 70-80°C. The solution was filtered at 40°C and the filtrate was allowed to stand in a refrigerator for 42 hours. The resulting crystals were collected by filtration and immediately dried under reduced pressure (37°C/5 mmHg, 8 hours) to give N-crystals of calcium gluconate (4.8 g). The powder X-ray diffraction pattern of the above N-crystals of calcium gluconate was in agreement with the pattern shown in Fig. 4.

Example 2

A 3-liter three-necked flask was filled with water (1200 ml) followed by addition of gluconodeltalactone - (178 g) and calcium carbonate (50 g). The mixture was stirred with heating at 95°C for 1 hour. The reaction mixture was cooled to 40°C and, then, filtered to give a clear calcium gluconate solution. This solution was cooled to 13-15°C, N-crystals of calcium gluconate (12 g) were added as seeds, and the mixture was stirred at that temperature for 2.5 hours. The resulting crystals were collected by filtration and dried under reduced pressure at room temperature (5 mmHg, 24 hours) to give N-crystals of calcium gluconate (130 g).

The powder X-ray diffraction pattern of the N-crystals of calcium gluconate thus obtained was in agreement with the pattern shown in Fig. 4.

Claims

1. A N-typed crystal of calcium gluconate having a powder X-ray diffraction pattern as shown in Fig. 4.

2. A process for preparation of a N-typed crystal of calcium gluconate having a powder X-ray diffraction pattern as shown in Fig. 4 which comprises precipitating calcium gluconate from an aqueous conventional calcium gluconate solution having the property that conventional crystals of calcium gluconate are not precipitated therefrom at around 40°C, at below 20°C.

3. A process for preparation of a N-typed crystal of calcium gluconate having a powder X-ray diffraction pattern as shown in Fig. 4 which comprises the following steps:

1) Gluconic acid or gluconodeltalactone is dissolved in water.

2) Calcium carbonate is added to the resulting solution and the solution is stirred with heating.

3) The reaction mixture is cooled to around 40°C and filtered. and

4) The filtrate is further cooled to about 13-15°C and N-typed crystals of calcium gluconate are added as seeds and the mixture is stirred at the same temperature for a few hours.

Fig. 1

Fig. 2

Fig. 3

Fig. 4

10°                    20°                    30° (2θ)

Fig. 5

Fig. 6.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | ULLMANNS ENCYKLOPÄDIE DER TECHNISCHEN CHEMIE, 3rd edition, vol. 9, 1957, page 673; Urban & Schwarzenberg, Munich<br>* page 673, line 10 * | 1 | C 07 C 59/105<br>B 01 D 9/02 |
| A | CHEMICAL ABSTRACTS, vol. 83, no. 14, 6th October 1975, page 431, column 1, abstract no. 120810z, Columbus, Ohio, US; B. JERZAK "Crystallization of calcium gluconate", & POL. 71, 433, 20-09-1974 (POLFA) | 1 | |
| A | CHEMICAL ABSTRACTS, vol. 102, no. 6, 11th February 1985, page 548, column 2, abstract no. 54115j, Columbus, Ohio, US; B.W. KROL "Crystallization of calcium gluconate", & PROCESS TECHNOL. PROC. 1984, 2(IND. CRYST.), 425-428 | 1 | TECHNICAL FIELDS SEARCHED (Int. Cl.4)<br><br>C 07 C 59/00<br>B 01 D 9/00 |

The present search report has been drawn up for all claims

| Place of search | Date of completion of the search | Examiner |
|---|---|---|
| BERLIN | 10-04-1987 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82